# EUROPEAN PATENT SPECIFICATION

(11) **EP 4 070 851 B1**
(45) Date of publication and mention of the grant of the patent: **20.09.2023**
(21) Application number: 22151438.3
(22) Date of filing: 13.01.2022
(51) Int. Cl.: A61N 1/36, A61N 1/372, G06F 3/01, A61N 1/05, A61B 5/00

(54) **AUTOCALIBRATION OF A COMPUTER BRAIN INTERFACE DEVICE**
AUTOKALIBRIERUNG VON EINER COMPUTER-GEHIRN-SCHNITTSTELLENVORRICHTUNG
AUTO-ÉTALONNAGE D'UN DISPOSITIF D'INTERFACE CERVEAU-ORDINATEUR

(30) Priority: 07.04.2021 US 202117224953
(43) Date of publication of application: 12.10.2022
(73) Proprietor: CereGate GmbH, 81671 München (DE)
(72) Inventor: HAGH GOOIE, Saman, 20457 Hamburg (DE); VÁRKUTI, Bálint, 81679 Munich (DE); SMITS SERENA, Ricardo, 80687 Munich (DE)
(74) Representative: Bardehle Pagenberg Partnerschaft mbB Patentanwälte Rechtsanwälte

(56) References cited:
- US-A1- 2013 253 299
- US-A1- 2017 080 226
- US-A1- 2018 229 046

## Description

### 1. Technical field

The present disclosure relates to a closed-loop autocalibration of a computer brain interface, CBI, device (and other types of neurostimulation equipment) that can be used to adjusts stimulation parameters of the CBI device to ensure - *inter alia -* robust and consistent information transfer from the CBI device to the brain with high fidelity.

### 2. Technical background

Several promising approaches for implementing a general-purpose CBI are based on implantable neurostimulation systems that typically include one or more neurostimulation electrodes implanted at a desired stimulation site within or close to the nervous system of a person. A neurostimulator (e.g., an implantable pulse generator (IPG)) typically generates neurostimulation signals that are then applied to the neurostimulation electrodes in order to elicit a neural response (e.g., action potentials) in specific parts of the nervous system. For instance, DE 10 2019/202666,

US 2020/0269049 and WO 2020/174051 describe such general-purpose CBI devices and systems that use direct neurostimulation of afferent sensory pathways to communicate abstract conceptual information directly to the brain of an individual.

For such CBI devices and systems to work reliably even in normally behaving (e.g. moving) individuals it has to be ensured that a given neurostimulation signal (or sequence of neurostimulation signals) that for instance is associated with a given piece / block of abstract information to be communicated consistently evokes essentially the same neural response in the brain or nervous system of the individual (e.g. a touch sensation in the left hand associated with a movement instruction or balance support cue etc.).

In this context, e.g., due to positional sensitivity a problem occurs, when neurostimulation electrodes that have initially been calibrated to elicit a certain neural response when being provided with a specific neurostimulation signal move relative to the stimulation target (e.g., afferent sensory axons / nerve fibers / neurons terminating in a desired sensory cortex area of the individual). For instance, such a situation may occur when during a movement (e.g., a person changing its body posture from standing to sitting or lying, a person bending over, coughing, etc.) the distance between a spinal cord stimulation electrode and the target nerve fibers in the spinal cord changes. As a result, the same neurostimulation signal that has previously been calibrated for a specific relative orientation and / or distance between electrode and spinal cord nerve fiber will not elicit the same desired neural response. In the prior art this distance is sometimes called dCSF. High bandwidth CBIs typically require complex and fine-tuned neurostimulation signals and thus are affected by such problems more severely than conventional neurostimulation equipment, e.g., for pain treatment etc.

Some aspects of the present disclosure relate to technical improvements / complements to the autocalibration method, device and system disclosed in US patent application 17/224,953.

In addition to the prior art already discussed in for US 17/224,953, US 11,045,129 relates to an implantable device for estimating neural recruitment arising from a stimulus that has a plurality of electrodes. A stimulus source provides stimuli to be delivered from the electrodes to neural tissue. Measurement circuitry obtains a measurement of a neural signal sensed at the electrodes. A control unit is configured to control application of a selected stimulus to neural tissue using the stimulus electrodes and after the selected neural stimulus, apply a probe stimulus having a short pulse width. A remnant neural response evoked by the probe stimulus is measured and the control unit estimates from the remnant neural response a neural recruitment caused by the selected neural stimulus.

Further, US 11,129,991 relates to a system configured to deliver electrical stimulation therapy to a patient, the electrical stimulation therapy comprising a plurality of therapy pulses at a predetermined pulse frequency over a period of time and deliver, over the period of time, a plurality of control pulses interleaved with at least some therapy pulses of the plurality of therapy pulses. The system may also be configured to sense, after one or more control pulses and prior to an immediately subsequent therapy pulse of the plurality of therapy pulses, a respective evoked compound action potential, ECAP, adjust, based on at least one respective ECAP, one or more parameter values that at least partially defines the plurality of therapy pulses, and deliver the electrical stimulation therapy to the patient according to the adjusted one or more parameter values.

US 11,129,987 relates to an Implantable Pulse Generator, IPG, or External Trial Stimulator, ETS, system that is capable of sensing an ECAP, and in conjunction with an external device is capable of adjusting a stimulation program while keeping a location of a Central Point of Stimulation, CPS, constant. Specifically, one or more features of measured ECAP(s) indicative of its shape and size are determined, and compared to thresholds or ranges to modify the electrode configuration of the stimulation program. US 2018/229046 A1 relates to communications along a neural pathway. The neural pathway is stimulated at a first location, in order to evoke neural responses which propagate along the neural pathway, the neural responses being modulated with data. At a second location spaced apart from the first location along the neural pathway the evoked neural responses are sensed. The sensed neural responses are then demodulated to retrieve the data. The stimulation could comprise peripheral sensory stimulation, and the second location could be at an implanted electrode array.

US 2017/080226 A1 refers to a system including a cochlear implant (CI) device and a screening device. The CI device includes an electrode array comprising a plurality of stimulation electrodes to be implanted in a patient's cochlea for electrical stimulation of the cochlea, a stimulation signal unit for generating stimulation signals to be supplied to the stimulation electrodes, and a unit for capturing ECAP signals induced at the electrodes in response to stimulation of the cochlea by applying stimulation signals to the electrodes. The screening device is adapted to cooperate with the stimulation signal unit in a manner so as to provide the electrodes with stimulation signals suitable for conducting growth function measurements, recovery measurements and spread-of-excitation measurements.

US 2013/253299 A1 relates to methods and apparatuses for monitoring and regulating physiological states and functions. Several embodiments include application of one or more microelectrode arrays to a dorsal root ganglion for measurement of sensory neuron activity, or stimulation of sensory reflex circuits. The methods and apparatuses can be used, for example, for monitoring or controlling bladder function in a patient.

Further prior art that forms general technical background of the present disclosure is provided by US 9,872,990, US 10,926,092, US 10,940,316, US 10,960,211.

### 3. Summary

The methods, devices and systems provided by the prior art have various deficiencies. For instance, they may not allow to perform closed-loop and on-line re-calibration of CBI stimulation parameters or only to a very limited extend. In addition, consistency and long-term stability and / or fidelity of desired artificial sensory perceptions / artificial sensations that are to be elicited in specific sensory cortex areas cannot be ensured with the prior art systems, mainly because in the prior art this technical problem faced by CBI devices does not even arise or does not have the same importance as it has for high bandwidth general purpose CBI applications. In essence, several of the prior art systems discussed above utilize closed-loop methods for detecting neural responses to minimize the occurrence of certain reactions / effects (such as paresthesias, pain etc.). To use an analogy, the prior art systems effectively function similar to audio speakers or headset systems that contain microphones which detect the emitted sound level and will automatically lower the volume if a threshold is reached, thereby protecting the user from unpleasant sensation / high volume sound. By contrast, the stringent fidelity requirements that need to be fulfilled in order to establish a general purpose and high-bandwidth CBI device are of a completely different quality and require a fundamentally different approach.

It is thus a problem underlying the present disclosure to overcome such deficiencies of previous technologies by providing novel closed-loop autocalibration of CBI devices and systems.

For instance, while prior art approaches typically involve comparing neural activity recordings (e.g., ECAP recordings) to a *"threshold"* or a *"threshold value"* and adjusting stimulation parameters based on determining whether such a threshold is crossed or not, the concept of the existence of a threshold is a simplification that, in some prior art application scenarios may still be sufficient e.g., for suppressing unwanted neural responses, e.g., for pain management or Parkinson Disease management devices.

However, stimulation parameter calibration methods based on this simplification may fail for advanced CBI-paradigms that involve highly consistent high-fidelity stimulation of artificial sensory perceptions as for instance disclosed in US 2020/0269049 and WO 2020/174051. To ensure CBI stimulation fidelity and consistency even in behaving individuals, the inventors of the present disclosure have found that it is instrumental to take into account the fundamental non-linear and dynamic nature of neural excitability when designing stimulation parameter feedback loops.

A key insight of the present disclosure is that already the scientific studies of Hodgkin and Huxley (Nobel prize in physiology 1963) showed that neurons are non-linear dynamical systems, and thus should be treated and interacted with as such, when designing CBI technology and in particular CBI parameter calibration methods and systems. In general, an afferent sensory neuron needs to be described by a set of dynamical variables that describe its state and a dynamical law that describes the evolution of the state variables with time (e.g., a set of coupled differential equations). For example, a proper dynamical description of an afferent sensory neuron may be based on variables describing neuronal dynamics such as a trans-membrane potential, an activation variable of Na+ currents (e.g., ion-channels), an inactivation variable of Na+ currents and activation variable of a fast K+ currents etc. as well as slowly varying adaptation variables, such as an activation of slow voltage- or Ca2+-dependent transmembrane currents. These adaptation variables may change during prolonged neurostimulation and can affect excitability on an intermediate or even on time scales much longer that the duration of a typical action potential and may even change the type of bifurcation behavior and /or phase space topology underlying the excitability of the stimulated afferent sensory neurons used for establishing the CBI channels to the brain.

Examples of the present disclosure allow to capture such complex dynamical system behavior of stimulated afferent sensory neurons and to use this information for enhanced closed-loop online calibration of CBI stimulation parameters. For reference, neuronal excitability is extensively reviewed in "Dynamical Systems in Neuroscience: The Geometry of Excitability and Bursting"; Eugene M. Izhikevich; The MIT Press, 2007.

Naturally, while discussed in the following with reference to a CBI device, the aspects of the present disclosure can also be applied to other types of neurostimulation devices, systems and equipment that might like-wise benefit from the present disclosure, such as DBS devices or spinal cord stimulation devices, etc. thar are applied for treating neurological conditions or are used for pain management. Thus, wherever the term "CBI" appears in the following it is to be understood that it also covers such other types of neurostimulation devices, systems and equipment.

The invention is defined by independent claims 1 and 14.

Preferred embodiments are defined in the dependent claims.

Importantly, as discussed in more detail below, the derivation of a dynamic excitability profile that is enabled by recording the dynamic bioelectric response associated with a burst sequence of stimulation pulses ensures that non-linear dynamical aspects such as sub-critical bifurcation characteristics, phase space topology as well as time scale separation effects, such as slowly varying (as compared to the action potential dynamics) physiologic adaptations modifying action potential dynamics on medium to long time scales can be appropriately taken into account when adjusting stimulation parameters of the CBI device. In this manner, it can be ensured that the CBI device consistently evokes essentially the same cortical response patterns needed for establishing and maintaining a high CBI channel bandwidth, sensory percept fidelity and information complexity that are all instrumental for general purpose CBI performance.

For instance, the burst sequence of stimulation pulses may part of a neurostimulation signal or a signal sequence, applied by the CBI device to elicit an artificial sensory perception in a sensory cortex area receiving input signals from a subset of the plurality of afferent sensory neurons (e.g., for communicating abstract semantic information or for high fidelity sensory substitution or enhancement). In this manner, for example, no dedicated calibration signals are required, and the excitability profile directly corresponds to the actual operation conditions / parameters of the CBI device.

Further, the burst sequence of stimulation pulses may comprise a burst sequence of essentially identical and / or phasic stimulation pulses. Further, the intra-burst pulse frequency may be at least 50 Hz, preferably at least 100 Hz, more preferably at least 200 Hz and even more preferably at least 250 Hz. For instance, using such pulse parameters allows to characterize the dynamic excitation behavior and certain phase-space properties such as a bifurcation type, phase-space topology, limit-cycle behavior etc. that otherwise could not be taken into account when adjusting stimulation parameters.

Further, a typical value for a burst repetition rate may be 1 to 10 Hz and for a pulse count within a burst sequence may be in the range of 3 to 50, preferably in the range of 5 to 25. For instance, to improve characterization of the non-linear excitability behavior of the stimulated neurons, intra-burst frequency should correspond to the refractory period of the neurons and / or burst repetition rate should allow to capture the slow dynamics of physiologic adaptation processes.

Further, recording the elicited dynamic bioelectric response may comprises recording the bioelectric response while the sequence of stimulation pulses is being applied, preferably after each stimulation pulse or continuously during the sequence and wherein deriving the excitability profile is based at least in part on intra-burst variations of the recorded bioelectric response. Further, a sampling rate of the recording may be larger or equal to the inverse of the time duration between two stimulation pulses of the sequence of stimulation pulses, preferably at least twice as large, more preferably at least 10 times as large and even more preferably at least 100 times as large. For example, preferably, the recording sampling rate may be at least 30 kHz or at least 100kHz.

Using such recoding techniques and recoding parameters further enhances how the dynamic excitation behavior of the stimulated plurality of afferent sensory neurons can be characterized.

Further, in particular for being more sensitive to slow physiologic adaptation effects and / or for deriving non-trivial properties of the phase space of the non-linear excitation dynamics, in some embodiments, at least two consecutive burst sequences may be applied, and deriving the excitability profile may be based at least in part on analyzing inter-burst variations of the recorded bioelectric response(s). Optionally, stimulation parameters such as intra-burst pulse frequency, amplitude, polarity, etc. may be varied among the at least two consecutive burst sequences.

For instance, varying the pulse frequency (e.g., of a sequence of individually sub-critical pulses) among consecutive burst sequences may allow to characterize non-linear dynamic phenomena such as non-linear resonances, phase-locking, synchronization, Arnold tongues, bifurcation types, etc.

For example, in some embodiments, deriving the dynamic excitability profile may be based at least in part of correlating the recorded bioelectric response with predictions of a non-linear mathematical model of neuronal excitability, comprising model parameters that vary slowly in time to capture physiologic adaptation mechanisms of the stimulated afferent sensory neurons.

For similar reasons as above, in some embodiments, at least two burst sequences may be applied and deriving the dynamic excitability profile may comprises analyzing the bioelectric response(s) corresponding to each stimulation pulse within a burst sequence as well as the joint or total bioelectric response corresponding to each burst sequence. Additionally of alternatively deriving the excitability profile may comprises analyzing variations among the recorded bioelectric responses within one burst sequence and / or among consecutive burst sequences.

To further improve, at least in some embodiments, the characterization of the non-linear excitability properties of the stimulated neurons, deriving the excitation profile may also comprise extracting temporal variations or dynamics of recording signal parameters or derived metrics from a plurality of subsequent recordings of the elicited bioelectric response and / or continuous recordings and / or classifying the excitation behavior of a subset of the stimulated afferent sensory neurons may use a closed set of discrete categories and based at least in part on the derived excitability profile.

For instance, such classification into excitability categories may be based on a metric (such as an absolute value or levels) for one or more signal parameters extracted from the recorded bioelectric responses. In addition, such classification may also be based on dynamical properties such as the type of bifurcation behavior (e.g., sub-critical Poincare-Andronov-Hopf bifurcation, saddle-node bifurcation etc.) underlying the non-linear behavior of the stimulated afferent sensory neurons. In other examples, such classification may also quantify in a discrete manner the distance (e.g., in phase space) from generating an action potential. Optionally, classification may also be based at least in part on analyzing a temporal variation or dynamic of the excitability profile within one burst sequence of stimulation pulses and / or among consecutive burst sequences of stimulation pulses.

In some aspects, the elicited bioelectric response(s) may comprise one or more compound action potentials, CAPs, and deriving the excitability profile may comprise determining one or more of: an N1 / P2 amplitude; a number of detectable peaks or minima, a measure of synchrony (in time) among the CAP responses within the sequence or among subsequent sequences of stimulation pulses and / or a delay between a stimulation pulse and the corresponding CAP response.

Further, adjusting the at least one stimulation parameter may comprise, at least in some embodiments, comparing the derived excitability profile with a reference excitability profile.

For example, the reference excitability profile may includes one or more of the following information: an amplitude of a reference bioelectric response, intra-burst variations among bioelectric responses corresponding to single stimulation pulses within a burst sequence, intra-burst variations of the bioelectric response corresponding to the first and the last stimulation pulse within a burst stimulation sequence and inter-burst variations of the bioelectric response.

As discussed in detail in 17/224,953 such a reference bioelectric response may be stored in a memory module of the CBI device or obtained via a communication interface of the CBI device. Accordingly, in some embodiments, the reference excitability profile may correspond to a specific artificial sensory perception corresponding to a set of reference stimulation parameters associated with the reference excitability profile.

Further, a neurostimulation signal or signal sequence may be applied to a subset of the afferent sensory neurons using the updated stimulation parameters wherein the neurostimulation signal may be configured to elicit an artificial sensory perception / percept in a sensory cortex area receiving afferent sensory input from the stimulated subset of afferent sensory neurons.

Further, in some embodiments, the calibration discussed above may incorporate other types physiological signals such as myogenic potentials or recordings from cortical areas as feedback information.

Further a computer brain interface, CBI, device is provided that comprises data and signal processing circuitry for carrying out the instructions of a computer program as discussed above.

In some aspects, such a CBI device may comprise one or more stimulation and sensing channels adapted to elicit and sense a bioelectric response of one or more afferent sensory neurons terminating (e.g. mono- or multi-synaptically) in a sensory cortex area. Such a CBI device may further comprise a memory module operably connected to the data and signal processing circuitry storing a first mapping between one or more artificial sensations that can be elicited by the CBI device in one or more sensory cortex areas of the individual and one or more bioelectric responses and / or storing a second mapping between a plurality of sets of neurostimulation signal parameters and a plurality of bioelectric responses of the one or more afferent sensory neurons (e.g. recorded upon initial calibration and / or during on-line recalibration as outline above).

### 4. Short Description of the Figures

Various aspects of the present disclosure are described in more detail in the following by reference to the accompanying figures. These figures show:
- **Fig. 1**: a diagram illustrating an individual being equipped with a CBI device according to aspects of the present disclosure;
- **Fig. 2**: a functional block circuit diagram illustrating a CBI device according to aspects of the present disclosure;
- **Fig. 3**: a diagram illustrating a set of dynamic bioelectric responses recorded from afferent sensory neurons according to aspects of the present disclosure.
- **Fig. 4**: a diagram illustrating examples of temporally varying excitability profiles according to some aspects of the present disclosure;
- **Fig. 5**: a diagram illustrating a basic example of intra-burst recording according to aspects of the present disclosure;
- **Fig. 6**: a diagram illustrating a basic example of inter-burst recording according to aspects of the present disclosure.

### 5. Detailed Description of some exemplary embodiments

In the following, some exemplary embodiments of the present invention are described in more detail, with reference to a CBI device that can be interfaced with neurostimulation electrodes such as spinal cord stimulation electrodes and / or DBS electrodes, e.g., via an intermediate neurostimulation device. However, the present disclosure can also be used with any other neurostimulation interface that is capable of stimulating afferent sensory neurons (e.g., axons, nerve fibers, etc.) of the central or peripheral nervous system targeting directly or indirectly a sensory cortex area of an individual.

While specific feature combinations are described in the following paragraphs with respect to the exemplary embodiments of the present disclosure, it is to be understood that not all features of the discussed embodiments have to be present for realizing the disclosure, which is defined by the subject matter of the claims. The disclosed embodiments may be modified by combining certain features of one embodiment with one or more technically and functionally compatible features of other embodiments. Specifically, the skilled person will understand that features, components and / or functional elements of one embodiment can be combined with technically compatible features, components and / or functional elements of any other embodiment of the present disclosure as long as covered by the invention specified by the appended claims.

Moreover, the various modules of the devices and systems disclosed herein can for instance be implemented in hardware, software or a combination thereof. For instance, the various modules of the devices and systems disclosed herein may be implemented via application specific hardware components such as application specific integrated circuits, ASICs, and / or field programmable gate arrays, FPGAs, and / or similar components and / or application specific software modules being executed on multi-purpose data and signal processing equipment such as CPUs, DSPs and / or systems on a chip, SOCs, or similar components or any combination thereof.

For instance, the various modules of the CBI devices discussed herein above may be implemented on a multi-purpose data and signal processing device (e.g., a smart phone) configured for executing application specific software modules and for communicating with various sensor devices and / or neurostimulation devices or systems via conventional wireless communication interfaces such as a NFC, a WIFI and / or a Bluetooth interface.

Alternatively, the various modules of the CBI devices discussed in the present application may also be part of an integrated neurostimulation apparatus, further comprising specialized electronic circuitry (e.g. neurostimulation signal generators, amplifiers etc.) for generating and applying the determined neurostimulation signals to a neurostimulation interface of the individual (e.g. a multi-contact electrode, a spinal cord stimulation electrode, peripheral sensory nerve stimulation electrode etc.) and for recoding the bioelectric responses as disclosed herein.

As discussed above the present disclosure may be realized in situations where the perceptual channels of a general-purpose CBI are not calibrated via subject-experimenter interactions. Instead, the CBI stimulation parameters can be self-calibrated by tapping into the neural activity of the tissue in vicinity of the stimulation interface. For instance, the level of induced bioelectric activation can be measured by interleaving recording bioelectric responses elicited by burst sequences of stimulation pulses.

In some examples, also described in 17/224,953 special test waveforms may be defined by modulating various aspect of the waveform in bursting mode. The modulated parameters of the waveform may include but are not limited to: a spatial activation pattern of the electrode contacts, an amplitude, an inter-pulse frequency, an inter-burst frequency, a pulse width, a wave-form shape (e.g. mono-phasic, biphasic with symmetric or with long active discharge period, multiphasic, etc.), a density of pulses within a burst or a burst duration. In an exemplary stimulation paradigm, a few symmetric pulses (e.g., in a range of 4 - 9 pulses) are delivered within short bursts (e.g., lasting 40 ms - 60 ms) to convey information related to intensity of sensation. The intensity can then be varied at a second measurement of loci point in time by changing density of pulses per burst while keeping pulse numbers constant i.e. shortening duration but increase intra-burst frequency and vice-versa.

For instance, neural recordings / sensing of bioelectric responses may take place by ramping stimulation signal bursts in repetition, aggregate frequency power pre- and post-pulse for each step of the ramp across repeated bursts then create differential response profile to pulses with varied intensity for the same purpose, so that the CBI device can estimate the neural excitation behavior of the stimulated afferent sensory nerve fibers by fitting a response function to the amplitude of the ECAP or theta frequency band of the ECAPs taking into account the response at every intensity increment. As stated above the excitation behavior can also be estimated not only by varying the amplitude of the burst in a ramp by also by changing other parameters of the stimulation such as frequency, pulse width, as well as the inter burst intervals, for example.

The estimated dynamic excitability profile then allows to determine optimal stimulation parameters which are adequate to generate desired level of activity in the target tissue thereby stabilizing the intensity, locus and / or quality of artificial sensory perceptions in the targeted sensory cortex area. This may be achieved, for example, by determining the highest value parameter coefficients which crucially contribute to determination of sensation intensity.

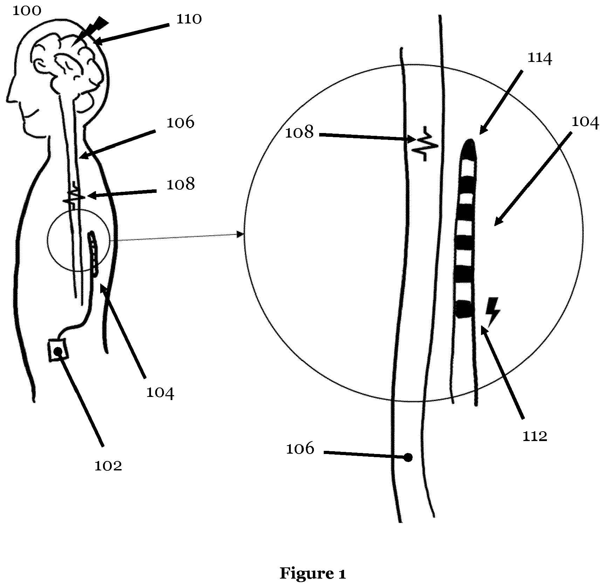

Figure 1 illustrates a person / individual 100 that is equipped with a CBI device as described in section 3 "Summary" above. In the illustrated embodiment, the CBI is implemented via direct neurostimulation of afferent sensory nerve fibers / neurons in the spinal cord 106 via one or more multi-contact electrodes 104 driven by an IPG 102 that may be operatively connected to or integrated with a CBI device as disclosed herein.

For establishing a perceptual communication channel to the brain of the individual 100 the CBI device typically is calibrated such that neurostimulation signals generated by the CBI device and applied via the IGP 102 and the multi-contact electrode 104 elicit one or more action potentials 108 in one or more afferent sensory nerve fibers of the spinal cord 106 targeting (e.g. via multi-synaptic afferent sensory pathways) one or more sensory cortex areas 110 of the individual where the one or more action potentials 108 generate artificial sensory perceptions that can be used to communicate with the individual 100. As discussed in detail in US 2020/0269049, fully incorporated herein by reference, artificial sensory perceptions that are elicited in a sensory cortex area (e.g., a sensory cortex area processing touch sensation on the left or right hand) can be associated with any kind of abstract information that is intelligible (i.e., consciously or subconsciously) by the individual.

While Fig. 1 shows orthodromically recoding, bioelectric responses may also be recorded differently, such as antiorthodromically.

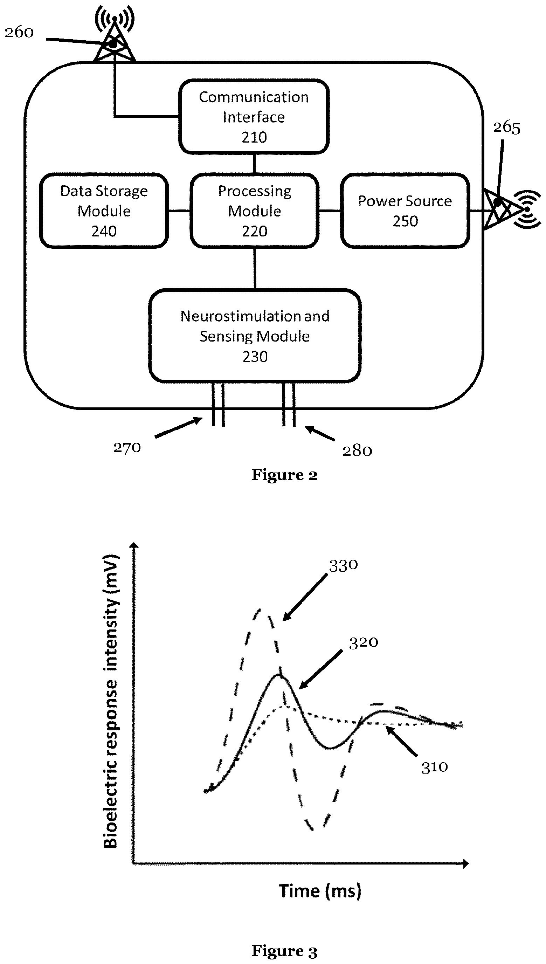

Figure 2 shows an exemplary CBI device according to an embodiment of the present invention. In this embodiment, the CBI device comprises an integrated neurostimulation and sensing module 230 (e.g. comprising a neuronal signal generator and an output amplifier as well as a sensing amplifier and an analog to digital converted) that is connected to a plurality of output signal leads 270 and a plurality of separate or identical sensing signal leads 280 that may be interfaced with a neurostimulation interface of the individual (e.g. a multi-contact spinal cord stimulation electrode such as the electrode 104 shown in Fig. 1). The CBI device may further comprise a communication antenna 260 operably connected to a communication interface module 210, configured for wireless communication (e.g., via NFC, Bluetooth, or a similar wireless communication technology).

The communication interface module 210 may be configured, for example, to receive one or more sensor signals from one or more sensors (not shown; e.g., acceleration signals obtained form an accelerometer etc.) and / or control information from a control device such as a remote control or a smart phone. The communication interface module 210 is operably connected to a data / signal processing module 220 configured to generate one or more neurostimulation signals and /or signal parameters (e.g., waveform, pulse shape, amplitude, frequency, burst count, burst duration etc.) for generating the one or more neurostimulation signals. For instance, the processing module 220 may access a data storage module 240 configured to store a plurality of relations, specific for the individual, associating a plurality of neurostimulation signals (or parameters used for generating a plurality of neurostimulation signals) with a plurality of corresponding pieces of information to be communicated to the individual.

The generated neurostimulation signals and / or the signal parameters are input into the integrated neurostimulation and sensing module 230 that may be configured to process (e.g., modulate, switch, amplify, covert, rectify, multiplex, phase shift, etc.) the one or more neurostimulation signals generated by the processing module 220 or to generate the one or more neurostimulation signals (e.g., burst sequences of stimulation pulses as discussed in the present disclosure) based on the signal parameters provided by the processing module 220.

The generated and processed neurostimulation signals are then output by the neurostimulation and sensing module 230 and can be applied to one or more electric contacts of a neurostimulation electrode (e.g., a DBS electrode or spinal cord stimulation electrode as shown in Fig. 1) via output leads 270. The CBI device of Fig. 2 may also comprise a rechargeable power source 250 that, for instance may be wirelessly charged via a wireless charging interface 265.

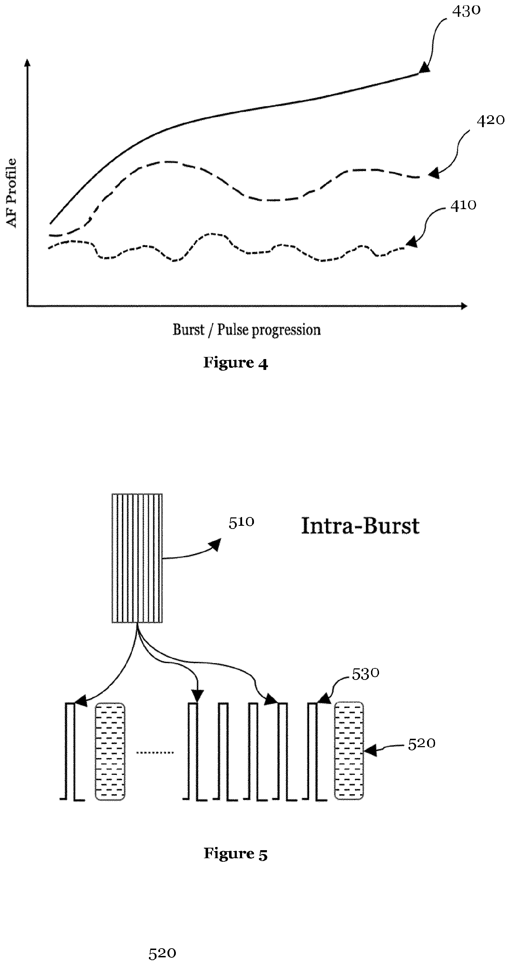

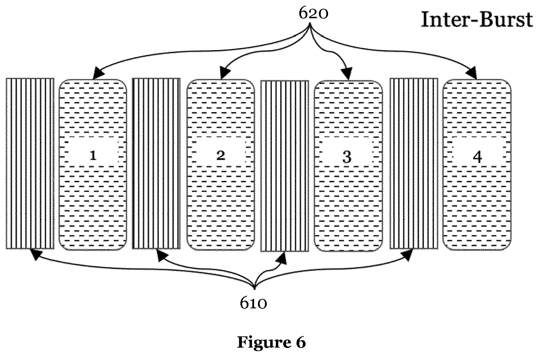

As discussed above, the data / signal processing module 220 may be further configured to, e.g., in conjunction with the data storage module 240 and the neurostimulation and sensing module 230, to execute a closed-loop, on-line autocalibration method as discussed and detail above and below. For example, it may generate one or more burst sequences of stimulation pulses (for examples see Fig. 5 and Fig. 6 below) configured to elicit a bioelectric response in one or more afferent sensory nerve fibers / neurons such as an evoked (compound) action potential in one or more afferent sensory nerve fibers / neurons of the spinal cord 106 as shown in Fig. **1****.**

The burst sequences of stimulation pulses may then be applied via output stimulation leads 270 to a neurostimulation interface such as the most caudal contact 112 of the multi-contact electrode 104 shown in Fig. **1****.** The neurostimulation and sensing module 230 may then sense, via the neurostimulation interface (e.g., via the most rostral contact 114), a bioelectric response 108 of the stimulated afferent sensory nerve fiber of the spinal cord 106.

Based on the sensed bioelectric response(s), the excitation behavior of the stimulated afferent sensory nerve fibers / neurons with respect to the neurostimulation interface can then be estimated by the neurostimulation and sensing module 230 and / or the processing module 220. As discussed above (e.g., see section 3 "summary"), based on the sensed bioelectric responses, a dynamic excitability profile can be derived and used for closed-loop stimulation parameter adaptation and / or stored in data storage module 240 for later use, e.g., for determining slowly varying physiologic adaptation processes as discussed above.

Figure 3 illustrates exemplary bioelectric responses 310, 320, 330 (e.g., extracellularly sensed (E)CAPs) of a sub-population of afferent sensory nerve fibers / neurons (e.g., of the spinal cord 106; see Fig. 1) sensed and recorded during application of a burst sequence of stimulation pulses according to aspects of the present disclosure (e.g., applied via the multi-contact spinal cord stimulation electrode 104 shown in Fig. 1) driven by a CBI device (see Fig. 2) according to an embodiment of the present disclosure. The illustrated bioelectric responses are sensed / recorded while several (e.g., consecutive) pulses within a burst sequence (see for example Fig. 5 below) are applied. Although the stimulation parameters for each pulse are kept constant, the bioelectric response changes substantially due to the non-linear nature of neuronal excitability as discussed above.

Based on such recordings of bioelectric responses the temporal dynamics of neuronal excitability can be derived and used for deriving the excitability profiles discussed in detail above.

Figure 4 illustrates three examples of such excitability profiles. On the x-axis pulse progression within a burst or several subsequent bursts is indicated. On the y-axis an excitation profile parameter such as the amplitude of the first peak or the difference of the second peak and the first valley or the delay between pulse and first peak or any other suitable recording signal parameter or metric as discussed above is plotted as function of burst progression.

The three exemplary traces 410, 420 and 430 may correspond to three different burst sequences each using different stimulation parameters. For instance, trace 410 may correspond to a set of parameters that do not result in (compound) action potential generation, trace 420 may correspond to a set of parameters that may result in inconsistent excitation behavior and trace 430 may correspond to a set of parameters that consistently evoke (compound) action potentials in a subset of the stimulated plurality of afferent sensory nerve fibers / neurons. In other situations, the three traces may also be recorded in subsequent stimulation trials with essentially identical pulse parameters, e.g., in situation where slow physiologic changes fundamentally shift the dynamic excitation behavior of the stimulated neurons.

As can be seen from Fig. 4 deriving a whole excitability profile is necessary to determine with high probability which set of stimulation parameters actually results in consistent action potential generation and thus should be used for operating the CBI device to transmit information to the brain. For instance, by just comparing the initial part of the three traces would not allow to characterize the excitation behavior and could thus result in wrongly adjusted stimulation parameters.

Auto-calibration of the perceptual channels of the CBI device can then be achieved by using a neural interface capable of stimulation and recording from the neural tissue. The derived excitability profiles and their dynamics may be compared after each individual stimulation pulse within a burst and / or between bursts in a trial to automatically determine the effectiveness of stimulation settings and establish various sensation levels within perceptual channels. Given similar stimulation parameters in each burst, the inter-burst dynamics of the excitability profile 430 exhibit a distinct shape compared to an undesired excitability profile as function of burst progression. It should be noted that although profile 420 may (locally) exhibit a higher intensity response, the stimulation cannot maintain an increasing profile evolution.

Figure 5 illustrates an intra-burst sequence recording configuration where the CBI device delivers (e.g., via a neurostimulation module; see Fig. 2) or commands an implanted stimulator to deliver a burst 510 of essentially identical stimulation pulses and records the induced bioelectric responses (e.g., action potentials, ECAPs, etc.) while the burst is applied. For instance, the induced bioelectric responses may be recorded 520 after the first and after the last stimulation pulse 530. In other embodiments, recordings may take place after each stimulation pulse within the burst or throughout in an essentially continuous manner (e.g., with a sampling rate of 100kHz) as discusses above.

Figure 6 illustrates an inter-burst sequence recording configuration where the CBI device delivers (e.g., via a neurostimulation module; see Fig. 2) or commands an implanted stimulator to deliver a sequence of bursts 610 of essentially identical stimulation pulses and records 620 the induced bioelectric responses (e.g., action potentials, ECAPs, resting potential, depolarization, etc.) after each burst sequence and optionally, also while each burst sequence is applied as illustrated in Fig. 5 and discussed above. Such a stimulation and recording sequence may enable the CBI to detect slowly varying variables that might affect neuronal excitability on medium to long time scales as also discussed above.

## Claims

1. A computer program for updating a current set of stimulation parameters of a neurostimulation, NS, device or a computer-brain interface, CBI, device, comprising instructions which, when being executed by data and signal processing circuitry of the NS device or the CBI device, cause the device to carry out the following steps:
applying, via a neurostimulation interface device (102, 104) operably connected to the CBI device or to the NS device, a burst sequence of stimulation pulses (510, 610) to a plurality of afferent sensory neurons (106) targeting a sensory cortex area (110) involved with decoding information transmitted by the NS or CBI device;
wherein the sequence of stimulation pulses is associated with the current set of stimulation parameters; and wherein the sequence of stimulation pulses is configured to elicit a bioelectric response (108) in the plurality of afferent sensory neurons;
recording, via the neurostimulation interface device, the elicited bioelectric response of the stimulated afferent sensory neurons;
deriving, based at least in part on the recorded bioelectric response, a neural excitability profile characterizing a non-linear, dynamic excitation behavior of the plurality of afferent sensory neurons corresponding to the applied sequence of stimulation pulses; and
adjusting, based on the derived excitability profile at least one stimulation parameter of the current set of stimulation parameters to obtain an updated set of stimulation parameters.

2. The computer program of claim 1, wherein the burst sequence of stimulation pulses is part of a neurostimulation signal or signal sequence, applied by the NS or CBI device to elicit an artificial sensory perception in a sensory cortex area receiving input signals from at least a subset of the plurality of afferent sensory neurons; and / or
wherein the burst sequence of stimulation pulses comprises a burst sequence of essentially identical and / or phasic stimulation pulses; and / or
wherein an intra-burst pulse frequency is at least 50 Hz, preferably at least 100 Hz, more preferably at least 200 Hz and even more preferably at least 250 Hz.

3. The computer program of claim 1 or 2, wherein the instructions for recording the elicited bioelectric response comprises instructions for recording the bioelectric response while the burst sequence of stimulation pulses is being applied, preferably after each stimulation pulse or continuously during the sequence; and wherein deriving the excitability profile is based at least in part on analyzing intra-burst variations of the recorded bioelectric response.

4. The computer program of any of the preceding claims, wherein a sampling rate of the recording is equal or larger than the inverse of a time duration between two stimulation pulses of the burst sequence of stimulation pulses, preferably at least twice as large, more preferably at least 10 times as large and even more preferably at least 100 times as large.

5. The computer program of any of the preceding claims, wherein at least two consecutive burst sequences are applied, and wherein deriving the excitability profile is based at least in part on analyzing inter-burst variations of the recorded bioelectric response; and optionally, wherein stimulation parameters, preferably the pulse frequency, are varied among the at least two consecutive burst sequences.

6. The computer program of any of the preceding claims,
wherein at least two burst sequences are applied; and wherein the instructions for deriving the excitability profile comprises instructions for analyzing the bioelectric response corresponding to each stimulation pulse within a burst sequence and the bioelectric response corresponding to each burst sequence; and / or
wherein the instructions for deriving the excitability profile comprises instructions for analyzing variations among the recorded bioelectric responses within one burst sequence and / or among consecutive burst sequences.

7. The computer program of any of the preceding claims, wherein the instructions for deriving the excitation profile comprises instructions for extracting temporal variations or dynamics of recording signal parameters or derived metrics from a plurality of subsequent recordings of the elicited bioelectric response and / or continuous recordings; and / or wherein the computer program further comprises instructions for classifying the excitation behavior of a subset of the stimulated afferent sensory neurons using a closed set of discrete categories and based at least in part on the derived excitability profile.

8. The computer program of claim 7, wherein classification is based at least in part on analyzing a temporal variation or dynamic of the excitability profile within one burst sequence of stimulation pulses and / or among consecutive burst sequences of stimulation pulses.

9. The computer program of any of the preceding claims, wherein deriving the excitability profile is based at least in part on correlating the recorded bioelectric response with predictions of a non-linear mathematical model of neuronal excitability, comprising model parameters that vary slowly in time to capture physiologic adaptation mechanisms of the stimulated afferent sensory neurons.

10. The computer program of any of the preceding claims, wherein the elicited bioelectric response comprises one or more compound action potentials, CAPs, and wherein deriving the excitability profile comprises determining one or more of: an N1 / P2 amplitude; a number of detectable peaks or troughs; a measure of synchrony among the bioelectric responses recorded for the burst sequence or among subsequent burst sequences of stimulation pulses, a delay between a stimulation pulse and the corresponding CAP.

11. The computer program of any of the preceding claims, wherein the instructions for adjusting the at least one stimulation parameter comprises instructions for comparing the derived excitability profile with a reference excitability profile,
wherein optionally, the reference excitability profile includes one or more of the following information: an amplitude of a reference bioelectric response, intra-burst variations among bioelectric responses corresponding to single stimulation pulses within a burst sequence; intra-burst variations of the bioelectric response corresponding to the first and the last stimulation pulse within a burst stimulation sequence and inter-burst variations of the bioelectric response.

12. The computer program of claim 11, wherein the reference excitability profile corresponds to a specific artificial sensory perception corresponding to a set of reference stimulation parameters associated with the reference excitability profile.

13. The computer program of any of the preceding claims, further comprising instructions for applying a neurostimulation signal or signal sequence to at least a subset of the afferent sensory neurons using the updated stimulation parameters wherein the neurostimulation signal or signal sequence is configured to elicit an artificial sensory perception in a sensory cortex area receiving afferent sensory input from the stimulated subset of afferent sensory neurons.

14. A neurostimulation, NS, or computer brain interface, CBI, device or system comprising:
data and signal processing circuitry operably connected to memory storing the computer program of claim 1; wherein the data and signal processing circuitry, when executing the instructions of the stored computer program, are configured to cause the NS or CBI device or system to:
apply, via a neurostimulation interface device (102, 104) operably connected to the CBI device or to the NS device, a burst sequence of stimulation pulses (510, 610) to a plurality of afferent sensory neurons (106) targeting a sensory cortex area (110) involved with decoding information transmitted by the NS or CBI device;
wherein the sequence of stimulation pulses is associated with the current set of stimulation parameters; and wherein the sequence of stimulation pulses is configured to elicit a bioelectric response (108) in the plurality of afferent sensory neurons;
record, via the neurostimulation interface device, the elicited bioelectric response of the stimulated afferent sensory neurons;
derive, based at least in part on the recorded bioelectric response, a neural excitability profile characterizing a non-linear, dynamic excitation behavior of the plurality of afferent sensory neurons corresponding to the applied sequence of stimulation pulses; and
adjust, based on the derived excitability profile at least one stimulation parameter of the current set of stimulation parameters to obtain an updated set of stimulation parameters.

15. The NS or CBI device or system of claim 14, wherein the memory stores the computer program of any of claims 2 to 13 and the data and signal processing circuitry are configured to cause the NS or CBI device or system to carry out the instructions of the stored computer program.

## Patentansprüche

1. Computerprogramm zum Aktualisieren eines aktuellen Satzes von Stimulationsparametern einer Neurostimulationsvorrichtung, NS-Vorrichtung, oder einer Computer-Gehirn-Schnittstellen-Vorrichtung, CBI-Vorrichtung, umfassend Anweisungen, die, wenn sie von einer Daten- und Signalverarbeitungsschaltung der NS-Vorrichtung oder der CBI-Vorrichtung ausgeführt werden, die Vorrichtung veranlassen, die folgenden Schritte auszuführen:
Anwenden, über eine Neurostimulationsschnittstellenvorrichtung (102, 104), die mit der CBI-Vorrichtung oder der NS-Vorrichtung wirkverbunden ist, einer Burst-Sequenz von Stimulationsimpulsen (510, 610) auf eine Mehrzahl von afferenten sensorischen Neuronen (106), die auf einen sensorischen Cortexbereich (110) abzielen, der an der Decodierung von Informationen beteiligt ist, die von der NS- oder CBI-Vorrichtung übertragen werden;
wobei die Sequenz von Stimulationsimpulsen mit dem aktuellen Satz von Stimulationsparametern konfiguriert ist; und wobei die Sequenz von Stimulationsimpulsen konfiguriert ist, um eine bioelektrische Antwort (108) in der Mehrzahl von afferenten sensorischen Neuronen auszulösen;
Aufzeichnen, über die Neurostimulationsschnittstellenvorrichtung, der ausgelösten bioelektrischen
Antwort der stimulierten afferenten sensorischen Neuronen;
Ableiten, zumindest teilweise basierend auf der aufgezeichneten bioelektrischen Antwort, eines neuronalen Erregungsfähigkeitsprofils, das ein nichtlineares, dynamisches Erregungsverhalten der Mehrzahl von afferenten sensorischen Neuronen entsprechend der angewendeten Sequenz von Stimulationsimpulsen charakterisiert; und
Anpassen, basierend auf dem abgeleiteten Erregungsfähigkeitsprofil, mindestens eines Stimulationsparameters des aktuellen Satzes von Stimulationsparametern, um einen aktualisierten Satz von Stimulationsparametern zu erhalten.

2. Computerprogramm nach Anspruch 1, wobei die Burst-Sequenz von Stimulationsimpulsen Teil eines Neurostimulationssignals oder einer Signalsequenz ist, die von der NS- oder CBI-Vorrichtung angewendet wird, um eine künstliche sensorische Wahrnehmung in einem sensorischen Cortexbereich auszulösen, der Eingangssignale von mindestens einer Teilmenge der Mehrzahl von afferenten sensorischen Neuronen empfängt; und/oder
wobei die Burst-Sequenz von Stimulationsimpulsen eine Burst-Sequenz von im Wesentlichen identischen und/oder phasischen Stimulationsimpulsen umfasst; und/oder
wobei eine Intraburst-Impulsfrequenz mindestens 50 Hz, vorzugsweise mindestens 100 Hz beträgt
mehr bevorzugt mindestens 200 Hz und noch mehr bevorzugt mindestens 250 Hz.

3. Computerprogramm nach Anspruch 1 oder 2, wobei die Anweisungen zum Aufzeichnen der ausgelösten bioelektrischen Antwort Anweisungen zum Aufzeichnen der bioelektrischen Antwort umfassen, während die Burst-Sequenz von Stimulationsimpulsen angewendet wird, vorzugsweise nach jedem Stimulationsimpuls oder kontinuierlich während der Sequenz; und wobei das Ableiten des Erregungsfähigkeitsprofils zumindest teilweise auf dem Analysieren von Intraburst-Variationen der aufgezeichneten bioelektrischen Antwort basiert.

4. Computerprogramm nach einem der vorhergehenden Ansprüche, wobei eine Abtastrate der Aufzeichnung gleich oder größer als der Kehrwert einer Zeitdauer zwischen zwei Stimulationsimpulsen der Burst-Sequenz von Stimulationsimpulsen ist, vorzugsweise mindestens doppelt so groß, mehr bevorzugt mindestens 10 mal so groß und noch mehr bevorzugt mindestens 100 mal so groß.

5. Computerprogramm nach einem der vorhergehenden Ansprüche, wobei mindestens zwei aufeinanderfolgende Burst-Sequenzen angewendet werden, und wobei das Ableiten des Erregungsfähigkeitsprofils zumindest teilweise auf dem Analysieren von Interburst-Variationen der aufgezeichneten bioelektrischen Antwort basiert; und wobei optional Stimulationsparameter, vorzugsweise die Impulsfrequenz, zwischen den mindestens zwei aufeinanderfolgenden Burst-Sequenzen variiert werden.

6. Computerprogramm nach einem der vorhergehenden Ansprüche,
wobei mindestens zwei Burst-Sequenzen angewendet werden; und wobei die Anweisungen zum Ableiten des Erregungsfähigkeitsprofils Anweisungen zum Analysieren der bioelektrischen Antwort, die jedem Stimulationsimpuls innerhalb einer Burst-Sequenz entspricht, und der bioelektrischen Antwort, die jeder Burst-Sequenz entspricht, umfassen; und/oder
wobei die Anweisungen zum Ableiten des Erregungsfähigkeitsprofils Anweisungen zum Analysieren von Variationen zwischen den aufgezeichneten bioelektrischen Antworten innerhalb einer Burst-Sequenz und/oder zwischen aufeinanderfolgenden Burst-Sequenzen umfassen.

7. Computerprogramm nach einem der vorhergehenden Ansprüche, wobei die Anweisungen zum Ableiten des Erregungsprofils Anweisungen zum Extrahieren von zeitlichen Variationen oder Dynamiken von Aufzeichnungssignalparametern oder abgeleiteten Metriken aus einer Mehrzahl von nachfolgenden Aufzeichnungen der ausgelösten bioelektrischen Antwort und/oder kontinuierlichen Aufzeichnungen umfassen; und/oder wobei das Computerprogramm ferner Anweisungen zum Klassifizieren des Erregungsverhaltens einer Teilmenge der stimulierten afferenten sensorischen Neuronen unter Verwendung eines geschlossenen Satzes von diskreten Kategorien und zumindest teilweise basierend auf dem abgeleiteten Erregungsfähigkeitsprofil umfasst.

8. Computerprogramm nach Anspruch 7, wobei die Klassifizierung zumindest teilweise auf dem Analysieren einer zeitlichen Variation oder Dynamik des Erregungsfähigkeitsprofils innerhalb einer Burst-Sequenz von Stimulationsimpulsen und/oder zwischen aufeinanderfolgenden Burst-Sequenzen von Stimulationsimpulsen basiert.

9. Computerprogramm nach einem der vorhergehenden Ansprüche, wobei das Ableiten des Erregungsfähigkeitsprofils zumindest teilweise auf dem Korrelieren der aufgezeichneten bioelektrischen Antwort mit Vorhersagen eines nichtlinearen mathematischen Modells der neuronalen Erregungsfähigkeit basiert, das Modellparameter umfasst, die zeitlich langsam variieren, um physiologische Anpassungsmechanismen der stimulierten afferenten sensorischen Neuronen zu erfassen.

10. Computerprogramm nach einem der vorhergehenden Ansprüche, wobei die ausgelöste bioelektrische Antwort ein oder mehrere Summenaktionspotentiale, CAPs, umfasst und wobei das Ableiten des Erregungsfähigkeitsprofils das Bestimmen von einem oder mehreren von Folgendem umfasst: einer N1 /P2 -Amplitude; einer Anzahl von detektierbaren Spitzen oder Tälern; einem Maß der Synchronie zwischen den bioelektrischen Antworten, die für die Burst-Sequenz oder zwischen nachfolgenden Burst-Sequenzen von Stimulationsimpulsen aufgezeichnet wurden, einer Verzögerung zwischen einem Stimulationsimpuls und dem entsprechenden CAP.

11. Computerprogramm nach einem der vorhergehenden Ansprüche, wobei die Anweisungen zum Anpassen des mindestens einen Stimulationsparameters Anweisungen zum Vergleichen des abgeleiteten Erregungsfähigkeitsprofils mit einem Referenzerregungsfähigkeitsprofil umfassen,
wobei optional das Referenzerregungsfähigkeitsprofil eine oder mehrere der folgenden Informationen beinhaltet: eine Amplitude einer bioelektrischen Referenzantwort, Intraburst-Variationen zwischen bioelektrischen Antworten, die einzelnen Stimulationsimpulsen innerhalb einer Burst-Sequenz entsprechen; Intraburst-Variationen der bioelektrischen Antwort, die dem ersten und dem letzten Stimulationsimpuls innerhalb einer Burst-Stimulationssequenz entsprechen, und Interburst-Variationen der bioelektrischen Antwort.

12. Computerprogramm nach Anspruch 11, wobei das Referenzerregungsfähigkeitsprofil einer spezifischen künstlichen sensorischen Wahrnehmung entspricht, die einem Satz von Referenzstimulationsparametern entspricht, die mit dem Referenzerregungsfähigkeitsprofil assoziiert sind.

13. Computerprogramm nach einem der vorhergehenden Ansprüche, ferner umfassend Anweisungen zum Anwenden eines Neurostimulationssignals oder einer Signalsequenz auf mindestens eine Teilmenge der afferenten sensorischen Neuronen unter Verwendung der aktualisierten Stimulationsparameter, wobei das Neurostimulationssignal oder die Signalsequenz konfiguriert ist, um eine künstliche sensorische Wahrnehmung in einem sensorischen Cortexbereich auszulösen, der afferente sensorische Eingaben von der stimulierten Teilmenge von afferenten sensorischen Neuronen empfängt.

14. Neurostimulationsvorrichtung, NS-Vorrichtung, oder Computer-Gehirn-Schnittstellen-Vorrichtung, CBI-Vorrichtung, oder -System, umfassend:
eine Daten- und Signalverarbeitungsschaltung, die mit einem Speicher wirkverbunden ist, der das Computerprogramm nach Anspruch 1 speichert; wobei die Daten- und Signalverarbeitungsschaltung, wenn sie die Anweisungen des gespeicherten Computerprogramms ausführt, konfiguriert sind, um die NS- oder CBI-Vorrichtung oder das -System zu Folgendem zu veranlassen:
Anwenden, über eine Neurostimulationsschnittstellenvorrichtung (102, 104), die mit der CBI-Vorrichtung oder der NS-Vorrichtung wirkverbunden ist, einer Burst-Sequenz von Stimulationsimpulsen (510, 610) auf eine Mehrzahl von afferenten sensorischen Neuronen (106), die auf einen sensorischen Cortexbereich (110) abzielen, der an der Decodierung von Informationen beteiligt ist, die von der NS- oder CBI-Vorrichtung übertragen werden;
wobei die Sequenz von Stimulationsimpulsen mit dem aktuellen Satz von Stimulationsparametern assoziiert ist; und wobei die Sequenz von Stimulationsimpulsen konfiguriert ist, um eine bioelektrische Antwort (108) in der Mehrzahl von afferenten sensorischen Neuronen auszulösen;
Aufzeichnen, über die Neurostimulationsschnittstellenvorrichtung, der ausgelösten
bioelektrischen Antwort der stimulierten afferenten sensorischen Neuronen;
Ableiten, zumindest teilweise basierend auf der aufgezeichneten bioelektrischen Antwort, eines neuronalen Erregungsfähigkeitsprofils, das ein nichtlineares, dynamisches Erregungsverhalten der Mehrzahl von afferenten sensorischen Neuronen entsprechend der angewendeten Sequenz von Stimulationsimpulsen charakterisiert; und
Anpassen, basierend auf dem abgeleiteten Erregungsfähigkeitsprofil, mindestens eines Stimulationsparameters des aktuellen Satzes von Stimulationsparametern, um einen aktualisierten Satz von Stimulationsparametern zu erhalten.

15. NS- oder CBI-Vorrichtung oder -System nach Anspruch 14, wobei der Speicher das Computerprogramm nach einem der Ansprüche 2 bis 13 speichert und die Daten- und Signalverarbeitungsschaltung konfiguriert sind, um die NS- oder CBI-Vorrichtung oder das -System zu veranlassen, die Anweisungen des gespeicherten Computerprogramms auszuführen.

## Revendications

1. Un programme informatique pour mettre à jour un ensemble courant de paramètres de stimulation d'un dispositif de neurostimulation, NS, ou d'un dispositif d'interfaçage calculateur-cerveau, CBI, comprenant des instructions qui, lorsqu'elles sont exécutées par une circuiterie de traitement de données et de signaux du dispositif NS ou du dispositif CBI, font en sorte que le dispositif effectue les étapes suivantes :
l'application, via une interface de neurostimulation (102, 104) reliée de manière opérante au dispositif CBI ou au dispositif NS, d'une séquence en salve d'impulsions de stimulation (510, 610) à une pluralité de neurones sensoriels afférents (106) ciblant une zone sensorielle du cortex (110) impliqué dans le décodage des informations transmises par le dispositif NS ou CBI ;
dans lequel la séquence d'impulsions de stimulation est associée à l'ensemble courant de paramètres de stimulation ; et dans lequel la séquence d'impulsions de stimulation est configurée pour induire une réponse bioélectrique (108) sur la pluralité de neurones sensoriels afférents ;
l'enregistrement, via le dispositif d'interfaçage de neurostimulation, de la réponse bioélectrique induite des neurones sensoriels afférents stimulés ;
la dérivation, au moins en partie sur la base de la réponse bioélectrique enregistrée, d'un profil d'excitabilité neuronale caractérisant un comportement non linéaire et dynamique à l'excitation pour la pluralité de neurones sensoriels afférents, correspondant à la séquence appliquée d'impulsions de stimulation ; et
l'ajustement, sur la base du profil d'excitabilité dérivé, d'au moins un paramètre de stimulation de l'ensemble courant de paramètres de stimulation pour obtenir un ensemble mis à jour de paramètres de stimulation.

2. Le programme informatique de la revendication 1, dans lequel la séquence en salve d'impulsions de stimulation fait partie d'un signal ou d'une séquence de signaux de neurostimulation, appliquée par le dispositif NS ou CBI pour induire une perception sensorielle artificielle dans une zone sensorielle du cortex recevant des signaux d'entrée depuis au moins un sous-ensemble de la pluralité de neurones sensoriels afférents ; et/ou
dans lequel la séquence en salve d'impulsions de stimulation comprend une séquence en salve d'impulsions de stimulation essentiellement identiques et/ou phasiques ;
et/ou dans lequel une fréquence des impulsions à l'intérieur d'une salve est d'au moins 50 Hz, de préférence d'au moins 100 Hz, plus préférentiellement d'au moins 200 Hz et encore plus préférentiellement d'au moins 250 Hz.

3. Le programme informatique de la revendication 1 ou 2, dans lequel les instructions pour l'enregistrement de la réponse bioélectrique induite comprennent des instructions pour enregistrer la réponse bioélectrique lorsque la séquence en salve d'impulsions de stimulation est en train d'être appliquée, de préférence après chaque impulsion de stimulation ou de façon continue durant la séquence ; et dans lequel la dérivation du profil d'excitabilité est au moins en partie basée sur l'analyse de variations à l'intérieur d'une salve de la réponse bioélectrique enregistrée.

4. Le programme informatique de l'une des revendications précédentes, dans lequel un taux d'échantillonnage de l'enregistrement est égal ou supérieur à l'inverse d'une durée d'un temps compris entre deux impulsions de stimulation de la séquence en salve d'impulsions de stimulation, de préférence au moins deux fois supérieur, plus préférentiellement au moins 10 fois supérieur et encore plus préférentiellement au moins 100 fois supérieur.

5. Le programme informatique de l'une des revendications précédentes, dans lequel au moins deux séquences consécutives en salve sont appliquées, et dans lequel la dérivation du profil d'excitabilité est au moins en partie basée sur l'analyse de variations entre salves de la réponse bioélectrique enregistrée ; et éventuellement dans lequel des paramètres de stimulation, de préférence la fréquence des impulsions, sont modifiées entre les au moins deux séquences en salve consécutives.

6. Le programme informatique de l'une des revendications précédentes,
dans lequel au moins deux séquences en salve sont appliquées ; et dans lequel les instructions pour dériver le profil d'excitabilité comprennent des instructions pour analyser la réponse bioélectrique correspondant à chaque impulsion de stimulation au sein d'une séquence en salve et la réponse bioélectrique correspondant à chaque séquence en salve ; et/ou
dans lequel les instructions pour dériver le profil d'excitabilité comprennent des instructions pour analyser des variations entre les réponses bioélectriques enregistrées au sein d'une même séquence en salve et/ou entre séquences en salve consécutives.

7. Le programme informatique de l'une des revendications précédentes, dans lequel les instructions pour dériver le profil d'excitabilité comprennent des instructions pour extraire des variations temporelles ou une dynamique de paramètres de signal d'enregistrement ou des métriques dérivées à partir d'une pluralité d'enregistrements ultérieurs de la réponse bioélectrique induite et/ou d'enregistrements en continu ; et/ou dans lequel le programme informatique comprend en outre des instructions pour classer le comportement à l'excitation d'un sous-ensemble des neurones sensoriels afférents stimulés en utilisant un ensemble fermé de catégories discrètes et au moins en partie sur la base du profil d'excitabilité dérivé.

8. Le programme informatique de la revendication 7, dans lequel la classification est au moins en partie basée sur l'analyse d'une variation temporelle ou d'une dynamique du profil d'excitabilité au sein d'une même séquence en salve d'impulsions de stimulation et/ou entre séquences en salve consécutives d'impulsions de stimulation.

9. Le programme informatique de l'une des revendications précédentes, dans lequel la dérivation du profil d'excitabilité est au moins en partie basée sur la corrélation de la réponse bioélectrique enregistrée avec des prédictions d'un modèle mathématique non linéaire d'excitabilité neuronale, comprenant des paramètres de modélisation qui varient lentement dans le temps pour capturer des mécanismes d'adaptation physiologique des neurones sensoriels afférents stimulés.

10. Le programme informatique de l'une des revendications précédentes, dans lequel la réponse bioélectrique induite comprend un ou plusieurs potentiels d'action composite, CAP, et dans lequel la dérivation du profil d'excitabilité comprend la détermination d'un ou plusieurs d'entre : une amplitude N1/P2, un nombre de pics ou de creux détectables ; une mesure de synchronisme entre les réponses bioélectriques enregistrées pour la séquence en salve ou entre séquences en salve ultérieures d'impulsions de stimulation ; un retard entre une impulsion de stimulation et le CAP correspondant.

11. Le programme informatique de l'une des revendications précédentes, dans lequel les instructions pour l'ajustement de l'au moins un paramètre de stimulation comprennent des instructions pour comparer le profil d'excitabilité dérivé à un profil d'excitabilité de référence,
éventuellement dans lequel le profil d'excitabilité de référence comprend une ou plusieurs des informations suivantes : une amplitude d'une réponse bioélectrique de référence, des variations à l'intérieur d'une salve entre réponses bioélectriques correspondant à des impulsions de stimulation uniques au sein d'une séquence en salve ; des variations à l'intérieur d'une salve de la réponse bioélectrique correspondant à la première et à la dernière impulsion de stimulation au sein d'une séquence de stimulation en salve ; et des variations entre salves de la réponse bioélectrique.

12. Le programme informatique de la revendication 11, dans lequel le profil d'excitabilité de référence correspond à une perception sensorielle artificielle spécifique correspondant à un ensemble de paramètres de stimulation de référence associés au profil d'excitabilité de référence.

13. Le programme informatique de l'une des revendications précédentes, comprenant en outre des instructions pour appliquer un signal ou une séquence de signaux de neurostimulation à au moins un sous-ensemble des neurones sensoriels afférents en utilisant les paramètres de stimulation mis à jour, le signal ou la séquence de signaux de neurostimulation étant configurés pour induire une perception sensorielle artificielle dans une zone sensorielle du cortex recevant une entrée sensorielle afférente en provenance du sous-ensemble stimulé de neurones sensoriels afférents.

14. Un dispositif ou système de neurostimulation, NS, ou d'interfaçage calculateur-cerveau, CBI, comprenant :
une circuiterie de traitement de données et de signaux reliée de manière opérante à une mémoire stockant le programme informatique de la revendication 1,
dans lequel la circuiterie de traitement de données et de signaux, lorsqu'elle exécute les instructions du programme de calculateur qui est stocké, sont configurées pour faire en sorte que le dispositif ou système NS ou CBI :
applique, via une interface de neurostimulation (102, 104) reliée de manière opérante au dispositif CBI ou au dispositif NS, une séquence en salve d'impulsions de stimulation (510, 610) à une pluralité de neurones sensoriels afférents (106) ciblant une zone sensorielle du cortex (110) impliqué dans le décodage des informations transmises par le dispositif NS ou CBI ;
dans lequel la séquence d'impulsions de stimulation est associée à l'ensemble courant de paramètres de stimulation ; et dans lequel la séquence d'impulsions de stimulation est configurée pour induire une réponse bioélectrique (108) sur la pluralité de neurones sensoriels afférents ;
enregistre, via le dispositif d'interfaçage de neurostimulation, la réponse bioélectrique induite des neurones sensoriels afférents stimulés ;
dérive, au moins en partie sur la base de la réponse bioélectrique enregistrée, un profil d'excitabilité neuronale caractérisant un comportement non linéaire et dynamique à l'excitation pour la pluralité de neurones sensoriels afférents, correspondant à la séquence appliquée d'impulsions de stimulation ; et
ajuste, sur la base du profil d'excitabilité dérivé, au moins un paramètre de stimulation de l'ensemble courant de paramètres de stimulation pour obtenir un ensemble mis à jour de paramètres de stimulation.

15. Le dispositif ou système NS ou CBI de la revendication 14, dans lequel la mémoire stocke le programme informatique de l'une des revendications 2 à 13, et la circuiterie de traitement de données et de signaux est configurée pour faire en sorte que le dispositif ou système NS ou CBI exécute les instructions du programme de calculateur qui est stocké.
